# EUROPEAN PATENT APPLICATION

(11) **EP 1 178 111 A1**
(43) Date of publication of application: **06.02.2002**
(21) Application number: 00202757.1
(22) Date of filing: 03.08.2000
(51) Int. Cl.: C12N 15/38, C12N 1/21, A61K 31/70, A61K 39/112, C12N 15/10

(54) **Vaccination against host cell-associated herpesviruses**

(71) Applicant: Lohmann Animal Health GmbH & Co. KG, 27472 Cuxhaven (DE)
(72) Inventor: Fehler, Frank, c/o Lohmann Animal Health GmbH & Co, 27454 Cuxhaven (DE); Osterrieder, Klaus, c/o Inst. of Molecular & Cel., 17498 Insel Riems (DE)
(74) Representative: Prins, Adrianus Willem

(57) **Abstract**

The invention relates to the field of so-called host cell-associated herpesviruses such as Marek's disease like virus (MDV) of poultry and of Varicella Zoster Virus (VZV) of man and to vaccination against disease caused by these viruses. The invention provides a vaccine directed against an infection caused by an herpesvirus that is essentially host cell associated comprising a recombinant viral genome derived from said herpesvirus, said genome allowing recombination essentially free of said host cell.

## Description

The invention relates to the field of vaccination against so-called host cell-associated herpesviruses such as Marek's disease virus (MDV) of poultry, and Varicella Zoster Virus (VZV, causing chickenpox and zoster after reactivation from latency) of man and to vaccination against disease caused by these viruses, and in particular relates to poultry disease in particular to the field of vaccination against Marek's disease.

In particular, Marek's disease has been a problem of the poultry industry from the beginning of intensive production of poultry meat. It is a herpesviral disease that is causing a large variety of clinical signs starting from immunosuppression, neurological disorders, anaemia and unspecified apathies and ending with severe lymphatic cancers at later stages of infection. In the beginning of the history of Marek's disease there were no treatments and no preventive measures. Then an apathogenic related (Serotype 3) virus was isolated from turkeys (HVT) and was initially used for vaccination.

However, some time after introduction of vaccination with HVT, Marek's disease emerged again and it became obvious, that the circulating field viruses had changed to circumvent the protection induced by the HVT-strain. At this time a new apathogenic virus was discovered (Rispens strain), which in general has the same serotype as the viruses causing disease. This vaccine strain was introduced very rapidly into the market and produced very good vaccination results.

However, again after about ten years new outbreaks of disease occurred, again circulating field viruses had changed to circumvent the protection induced by the vaccine-strain in current use. Then a combination of both vaccines (HVT and Rispens) was used to protect the animals, however, satisfactory results were only seen temporarily. Currently, new outbreaks of disease occur despite all these vaccinations. The reason for this is not yet understood but there is a clear need for the introduction of new potent vaccines.

Problems associated with vaccinations against Marek's disease are that despite the fact, that Marek vaccines have been produced for a long period, the method of preparation of the vaccines could not be improved. The reason for this is, that in general the essentially host cell-associated virus can be grown essentially only in primary host cells such as in the case of MDV or HVT in primary cells such as fibroblasts prepared from poultry, such as chickens, free of pathogens and in the case of Varicella Zoster Virus in (essentially primary) human cells (again, of course free of contaminating pathogens) and cannot or only with great difficulties be achieved out of context of the specific cell of the respective host. This makes in general a vaccine directed against viral infections or disease caused by these types of viruses difficult, if not nearly impossible on a practical level, to produce and thus expensive.

For example, the Rispens vaccine directed against Marek's disease, which is at present considered the only sufficiently potent one, is as all serotype-1 Marek viruses strictly host cell associated. Infectivity of the cell-associated virus (such as for example serotype 1 and 2) is completely lost during normal freezing or lyophilisation. Therefore the preparation of the vaccine includes a very complicated and expensive step, where whole cells must be frozen in liquid nitrogen. The vaccine must be stored and transported and kept under liquid nitrogen until use and therefore is causing tremendous costs and problems during transport.

Then, at the site of use, the vaccine must be used very carefully, since the infected cells are very sensitive to environmental factors. Factors such as elevated temperatures, exposure to light and residual detergents in used glassware often damage the virus such that no sufficiently viable vaccine batch can be prepared, leading to complete vaccine failures. Such failures can be recognised only when the disease already starts to break out and the affected poultry show symptoms of disease.

In short, up to now all attempts to provide inactivated -, subunit- or recombinant vaccines to protect against Marek's disease failed and therefore there is currently no alternative to live, cell-associated vaccines comprising Marek's Disease Virus. Marek's disease remains to be controlled by application of infected-cell preparations as vaccine. These preparations not only contain living cells suspended in DMSO-containing media and the whole variety of cellular antigens, they also have to be stored in liquid nitrogen. Consequently, the cold chain has to be maintained from vaccine production to the vaccine user and until administration. In addition, once thawed, the vaccine has to be administered within a very short period of time and every bird has to be injected. Several of these problems are shared by those wishing to prepare vaccines against other essentially cell associated Herpesviruses such as Varicella Zoster Virus.

Marek's disease virus (MDV) is a member of the *Alphaherpesvirinae* subfamily of the *Herpesviridae* (Lee et al, 2000, Murphy et al., 1995). Based on virulence for the chicken and ability to induce T cell lymphomas, MDV are generally grouped into three serotypes (MDV-1, MDV-2, and MDV-3). MDV-3 represents the herpesvirus of turkeys (HVT) which was widely used for vaccination against MDV-related disease. However, after vaccination failures and development of so-called virulent or very virulent MDV-1 (Witter, 1985), attenuated MDV-2 strains and later attenuated MDV-1 strains (e.g. strain CVI 988 Rispens) were used in vaccine formulations (Witter, 1985). In recent years and first reported in the United States, even more virulent MDV-1, so-called very virulent plus (vv+), MDV-1 variants appeared and caused high incidence of Marek's disease and mortality caused by tumour development and immunosuppression early after infection (Witter, 1997). One vv+ strain, 584A, was passaged more than 100 times on chicken embryo fibroblasts (CEF) and was shown to loose pathogenicity for chickens (Witter, 1997). However, the molecular basis for the increased pathogenicity of vv+ MDV-1 and similarly for loss of virulence are poorly understood because molecular analyses of MDV-1 are difficult to perform. On the one hand, no or only small amounts of infectious virus progeny is released in cultured cells, on the other hand production of MDV-1 recombinants is laborious and due to the highly cell-associated nature of the agent in cell culture, multiple rounds of purification of virus recombinants are needed (Cantello et al., 1991; Sakaguchi et al., 1993; Parcells et al., 1994; Schat et al., 1998; Anderson et al., 1998).

On top of that, as mentioned already above, vaccination can not guarantee to protect the animals from all Marek's disease field viruses. The virus - as all Herpesviruses - is capable of finding ways to escape the immune response induced by the vaccines. Therefore rapid adaptation of vaccines to the field situation would be needed. Currently, this is done by isolation of field isolates (such as HVT or Rispens) and/or further attenuation in vitro. The isolation itself is causing tremendous problems because of the difficulties of getting the cell-associated infectious virus out of chickens and infecting cells in cell-culture. The attenuation steps that would follow are very laborious and time consuming especially since plaque purification is extremely difficult, which is again due to the cell associated nature of the virus.

The result from attenuation is normally not defined. As a result of these facts no vaccines that would provide relief where current HVT- and Rispens-type vaccines fail have entered the market for a long time. In addition often an over-attenuation occurs during vaccine production since the virus has been passaged for too many times. This further aggravates the low efficacy of the HVT- and Rispens-type vaccines in the field. In short, the following problems constitute a large part of the current impasse in MDV control. There is a low reproducibility of classical vaccine production, one sees over-attenuation of vaccine virus, undefined attenuation of vaccine virus, high production costs, high storage and transport costs, high sensitivity of the vaccine to environmental factors, and a too slow development of new vaccine strains especially for cell associated viruses.

These problems are compounded by the fact that now circulating field viruses give rise to high antibody titres in poultry production stock, whereby these high antibody titres are given through the progeny via maternal antibody in the eggs. The influence of these maternal antibodies during initial infection by current vaccine virus further decrease the current efficacy of vaccination against Marek's disease.

The invention provides a vaccine directed against an infection caused by an herpesvirus that is essentially host cell associated comprising a recombinant viral genome derived from said herpesvirus, said genome allowing recombination essentially free of said host cell. To that effect, the invention herewith provides a recombinant viral genome derived from an herpesvirus that is considered to be essentially host cell associated, said genome preferably capable of at least some measure of replication in said host cell and at the same time allowing recombination essentially free or independently of said host cell, homologous recombination in eukaryotic cells is no longer required. In the detailed description such a genome is provided for a Marek's disease like virus.

Therein, as an example a genome of Marek's disease virus serotype 1 (MDV-1), strain 584Ap80C, was cloned in *Escherichia coli* as a bacterial artificial chromosome (BAC). BAC vector sequences were introduced into the Us2 locus of the MDV-1 genome by homologous recombination after cotransfection of chicken embryo fibroblasts (CEF) with viral DNA and recombinant plasmid pDS-pHA1 which contained BAC sequences and the *Eco-gpt* gene instead of the MDV-1 Us2 gene and flanking sequences. Transfection progeny was passaged on CEF cells in the presence of mycophenolic acid and xanthine/hypoxanthine. After four rounds of selection, viral DNA was prepared and used to transform *Escherichia coli* strain DH10B. Several colonies harboring the complete MDV-1 genome were identified. These MDV-1 BACs were transfected into CEF cells and from 3 days after transfection, infectious MDV-1 was recovered. Growth of MDV-1 recovered from various BACs was indistinguishable of that of the parental virus as assayed by plaque formation and determination of growth curves.

The invention thus provides a method for producing or obtaining a recombinant essentially host-cell associated herpesviral genome comprising (if required near complete or complete) infectious herpesviral nucleic acid derived from a MDV and/or VZV isolate.

Of course, now that the essentially complete genome is obtained free of the host cells it was originally thought to be firmly associated, the invention also provides a genome according to the invention that allows full application of all recombinant techniques available to the person skilled in the art of molecular biology, it for example thus also provides a vaccine according to the invention at least comprising a (replicative) minigenome.

For example, the invention provides a minigenome that provides for the expression of only a couple of glycoproteins (such as gB, gC, gD or combinations thereof) and e.g. ICP4 or another gene product that has been shown to induce cellular immunity in herpesviruses. Such a minigenome for example serves to identify genes, that are important in protection, considering that replication of the genome in (eukaryotic) host cells is no longer provided. Adding for example a HCMV or SV40 promotor in front of each gene or gene construct would provide for the final identification of a minimal protective unit. For a replication-competent minigenome the invention also provides deleting the entire, or a major part of the US region, whereby the resulting minivirus replicates also in host cells.

In another embodiment, the invention provides such a genome which comprises an essentially full-length copy derived from said herpesvirus, essentially full-length herein indicating that a great part of the genes of said viral genome is present, except for some that are preferably (at least functionally) left out such as a gene essential for replication or spread of the virus in a host or host cell culture, as provided herein in the detailed description. Therein, for example, in one of the recovered genomes according to the invention, BAC20, sequences encoding glycoprotein B (gB) were deleted by one-step recE-mediated mutagenesis using a linear DNA fragment. Glycoprotein B-negative MDV-1 reconstituted after transfection of gB-negative BAC20 DNA (20DgB) were only able to grow on cells providing gB *in trans*, demonstrating that gB is essential for MDV-1 growth in cultured host cells. Other genes essential for growth, and for which cells can be provided which produce the gene product *in trans* are gH, ICP4, UL15, UL28 and UL9, or another gene considered essential for growth such as listed below.

Furthermore, the invention provides use of a genome according to the invention for the preparation of a vaccine, in one embodiment such a vaccine directed against a disease caused by an infection with an essentially host cell-associated herpesvirus, however, in another embodiment such a vaccine may be used as vector vaccine and may comprise other or additional pathogens or nucleic acid stretches encoding therefor. For MDV, preferred additional pathogen nucleic acid comprises nucleic acid derived from for example Newcastle Disease virus, Eimeria spp, Salmonella spp, chicken infectious anaemia virus, influenza virus, infectious bursal disease virus, reovirus, or other pathogens commonly seen in poultry.

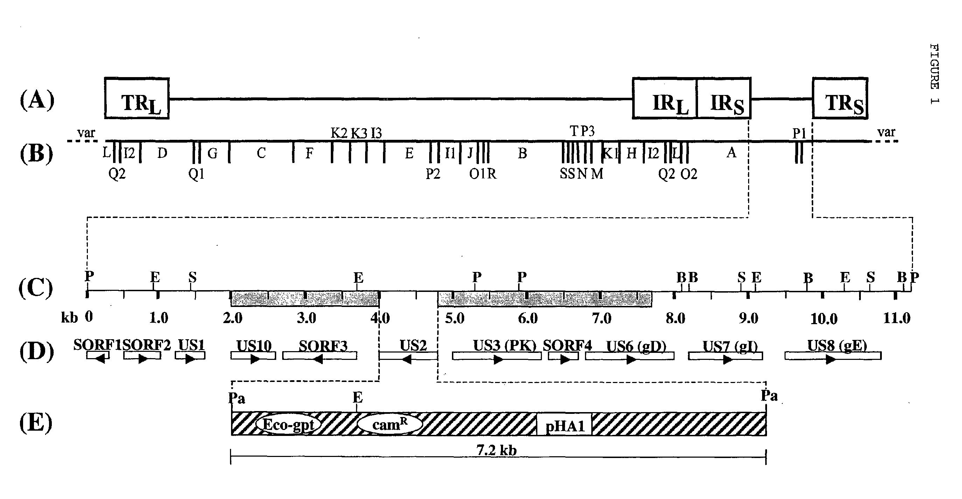

Thus, the invention also provides a vaccine wherein said genome comprises a functional deletion in a gene essential for replication and/or spread of said herpesvirus in a host cell, or wherein said viral genome at least comprises a nucleic acid encoding an antigenic substance capable of eliciting an (preferably essentially protective) immune response against an infection of an individual with said herpesvirus. An typical essential gene or fragment thereof to be deleted can for example be an MDV homologue of UL1 = glycoprotein L; ULS; UL8; UL9; UL15; UL18; UL19; UL22 = glycoprotein H; UL26; UL26.5; UL27 = glycoprotein B; UL28; UL29; UL30; UL52; UL53; ICP4 or genes or fragments thereof selected from the US region of the genome (figure 1).

In a preferred embodiment, the invention provides a vaccine comprising a functional deletion in a gene essential for eliciting a marker immune response specific for said herpesvirus allowing immunological discrimination between an individual vaccinated with said vaccine and an individual infected with said essentially cell associated herpesvirus. Preferred marker responses are for example directed at gC, gM, gD, or gE, whereby the detailed description further explains (here in the case of gM) such a deletion in a gene essential for eliciting a marker immune response.

Furthermore, the invention provides a vaccine according to the invention wherein said viral genome at least comprises a nucleic acid encoding a proteinaceous substance capable of modulating transcription and/or translation of a nucleic acid encoding an antigenic substance capable of eliciting an immune response against an infection of an individual with said herpesvirus.

Preferably, said vaccine comprises an essentially full-length copy derived from said herpesvirus, to maintain as many functions required for effective modulation of transcription and/or translation of the vaccine genome in the vaccinated host, however, minigenomic vaccination is also provided herein. It is of course preferred to efficiently modulate transcription and/or translation of a nucleic acid encoding a foreign pathogen, or antigenic substance derived thereof, when expressing an additional pathogen or an antigenic substance derived thereof from said genome, and it may be contemplated that also foreign (i.e. non-herpesvirus regulatory elements) are provided to said genome when providing a vaccine according to the invention provided with a nucleic acid encoding an additional pathogen.

In particular, the invention provides a vaccine according to the invention wherein said herpesvirus comprises Marek's disease-like virus. In particularly is it preferred to provide a vaccine wherein said Marek's disease-like virus comprises serotype 1. Also, now that methods for manipulation of the genome involved beyond the context of the host cell with which the genome originally was associated are provided, a vaccine is provided that, instead of being derived from normal attenuated or a-virulent isolates of Marek's disease-like virus, is derived instead from a virulent, a very virulent or a very virulent plus field-virus, because rapid isolation of infectious clones from field isolates is now possible, allowing preparation of DNA vaccines for prevention for Marek's disease vaccines in chicken and turkeys, where mutations into the genome very rapidly can be introduced. The same sytem can be used also for other essentially cell associated Herpesviruses like Varicella Zoster Virus.

The use of replicative viral genomes as provided herein containing parts of or entire and infectious Marek's Disease virus (MDV-1) genomes opens a variety of new possibilities to generate more efficacious, biologically safe and stable MDV-1 vaccines. Owing to the fact that recombinant MDV-1 are recovered from cloned DNA, virus progeny resulting from DNA transfections can be better characterized and 'over-attenuation' of vaccine viruses can be avoided. E.g., the number of 132-bp repeats which appear to be associated with attenuation (Maotani et al., 1986) can be exactly determined and - if necessary - be reduced or enlarged according to the needs for vaccine production or the situation in the field (see below). The generation of mutant MDV-1 is greatly facilitated. So far, MDV-1 mutants are generated by laborious and time-consuming homologous recombination and selection procedures in eukaryotic cells. These selection procedures - as reported for other herpesviruses - often result in mutations of the genome other than those desired, especially since in case of MDV-1 no cell-free virus can be obtained which makes selection procedures and recovery and propagation of mutants even more complicated. In contrast, the invention provides a method to manipulate a viral genome based on mutagenesis via a recE, recT and the recB/C-suppressing λ gam gene present on plasmid pGETrec (Narayanan et al., 1999). The advantages of the system are (i) that only 30 to 50 bp homology arms are needed to target a specific sequence to be deleted, i.e. deletion of any open reading frame can be achieved without the need to clone recombination cassettes, (ii) the method is very fast, and (iii) that the pGETrec vector conferring the mutagenesis system and expressing ampicillin resistance is rapidly lost from bacterial cells in the absence of ampicillin.

By using the powerful techniques using the so-called E/T cloning procedures, one-step mutation and selection in Escherichia coli is possible (Muyrers et al., 1999; Narayanan et al., 1999; Zhang et al., 1998). This technique also allows the deletion of essential MDV-1 genes without the need of using complementing cell lines since replication of mutated MDV-1 genomes as provided herein do not require the trans-complementation of the essential gene deleted. In addition, cloning procedures are completely unnecessary.

In another embodiment, the invention provides a method of generating MDV-1 or other (essentially cell-associated herpes) virus BACs, comprising transforming for example *Escherichia coli* DH10B cells with plasmid pBADαβγ, pGETrec or any other plasmid that inducibly or stably expresses *recE*, *recT* and the λ *gam* gene, followed by preparing circular viral DNA from lytically or latently infected cells taken for example *ex vivo* or from cell cultures. In a parallel or separate procedure, linear DNA harboring BAC vector sequences and sequences that allow homologous recombination of the BAC vector sequences with the viral DNA are provided. This linear DNA can e.g. be produced by PCR or by linearizing plasmid DNA. Then expression of *recE*, *recT* and the *gam* gene in the *Escherichia coli* is provided and electrocompetent cells are provided (e.g. Sambrook et al., 1989). Viral DNA is then electroporated together with linear DNA harboring the BAC vector sequences into competent *Escherichia coli.* Plating on agar containing appropriate antibiotics provides for the to be harvested colony or colonies and BAC DNA can be prepared as for example described in the detailed description herein. Infectivity of cloned viral BAC DNA is checked by transfection of susceptible cells. Herewith the invention provides a method to genetically recombine a essentially host-cell associated herpesviral genome derived from a host cell or tissue without the need to perform (homologous) recombination in eukaryotic cells allowing one to obtain an (if required near complete or complete) infectious genome or herpesviral nucleic acid derived from field-isolates or attenuated isolates alike.

The method as provided herein will also allow to further attenuate candidate vaccine MDV-1 or to generate MDV-1 mutants harbouring genes of other important chicken pathogens. In addition, emerging field MDV-1 isolates with possibly different and changing antigenetic properties can be countered by providing a vaccine based on exchange of the respective mutated genes between the cloned MDV-1 and the current field isolate(s). These changes - as described above - can be performed with the same E/T cloning technique and as such provide the possibility to react to changes of MDV-1 in the field very rapidly. An attractive advantage of a recovery of infectious MDV-1 from as described herein, however, is the use of a genome as provided herein as DNA vaccine. Up to now, Marek's disease is controlled by application of infected-cell preparations.

These preparations not only contain living cells suspended in DMSO-containing media and the whole variety of cellular antigens, they also have to be stored in liquid nitrogen. Consequently, the cold chain has to be maintained from vaccine production to the vaccine user and until administration. In addition, once thawed, the vaccine has to be administered within a very short period of time and every bird has to be injected. The invention provides use of a genome or a bacterium comprising said genome according to the invention for the preparation of a vaccine directed against a disease caused by an infection with an essentially host cell-associated herpesvirus. For example, with a MDV-1 genome DNA as provide herein, purification of the 'vaccine' (DNA) is easily feasible and reproducible. DNA is extremely stable, the maintenance of the cold chain is not necessary, and infectious DNA can be administered by several routes (intramuscularly, intradermally, in-ovo, orally, by the respiratory route, and so on) and in different formulations (with and without carrier etc.), leading to the expression of the desired vaccine virus or components thereof. In addition, the presence of maternal antibodies does not interfere with primary injection of the immunogen.

Beyond the possible vaccination with the MDV genome or VZV genome DNA as provided herein the invention also provides vaccination with an essentially intracellular bacterium comprising a genome according to the invention. Such bacteria, as provided herein, especially when being essentially intracellular or can invade cells (beyond being cultivable on normal bacterial culture media) release their DNA after the host has been, so to speak vaccinated with an essentially intracellular bacterium comprising a genome according to the invention, and release thus also the DNA as provided by the invention, during the course of infection or during recovery of the mucosa, thereby inducing replication of the desired vaccine virus within the host or vaccinate. Again DNA is expressed within the host cells and give rise to vaccine virus or at least parts thereof that induce the desired immune response within the target host or vaccinate. At least for chickens a preferred bacterium according to the invention comprises a *Salmonella sp,* preferably an attenuated *Salmonella typhimurium* or *Salmonella enteritidis* or other suitable attenuated Salmonella strain or isolate that can be used for vaccination purposes. Preferably, such strains still show invasiveness in chickens.

As such, MDV-1 genomes as herein provided allow for the first time the possibility to produce and engineer highly efficient and biologically safe vaccines against a tumorigenic and economically important disease. The invention thus in general provides method for limiting the risks of an individual on acquiring or fully manifesting a disease caused by an infection with an essentially host cell-associated herpesvirus comprising administering to said individual a vaccine according to the invention or a genome according to the invention.

### Detailed description:

Marek's disease virus (MDV) is a member of the *Alphaherpesvirinae* subfamily of the *Herpesviridae* (van Regenmortel et al., 1999). Based on virulence for chickens, ability to induce T cell lymphomas and antigenic properties, MDV are grouped into three serotypes (MDV-1, MDV-2, and MDV-3) (Payne, 1985). MDV-3 represents the herpesvirus of turkeys (HVT) which has been widely used for vaccination against MDV-related disease. According to the most recent nomenclature MDV-1 is classified as gallid herpesvirus 2 (GHV-2), MDV-2 as GHV-3, and HVT as meleagrid herpesvirus. All three viruses belong to the new *Marek's disease-like viruses* genus within the *Alphaherpesuirinae.*

Control of MDV-1 infection was achieved by vaccination primarily with HVT, however, after vaccination failures and description of so-called "very virulent" MDV-1 (Witter, 1989), MDV-2 strains and later attenuated MDV-1 strains (e.g. strain CVI 988 Rispens) have been used in vaccine formulations (Witter, 1985).

In recent years and first reported in the United States, even more virulent MDV-1, "very virulent plus" (vv+), MDV-1 variants appeared and caused high mortality even in vaccinated flocks (Witter, 1997). One of these vv+ strains, 584A, was passaged serially on chicken embryo fibroblasts (CEF) and lost pathogenicity for chickens (Witter, 1997). The molecular basis for the increased pathogenicity of vv+ MDV-1 and similarly for loss of virulence are poorly understood because molecular analyses of MDV-1 are difficult to perform.

On the one hand, no infectious virus progeny is released in cultured cells, on the other hand production of MDV-1 recombinants is laborious and due to the highly cell-associated nature of the agent *in vitro,* multiple rounds of purification of virus recombinants are needed (Cantello et al., 1991; Sakaguchi et al., 1993; Parcells et al., 1994,1995; Schat et al., 1998; Anderson et al., 1998). In addition, primary cells have to be used for growth of MDV-1 (Payne), resulting in the fact that analysis of essential MDV-1 genes is almost impossible because no trans-complementing cell lines can be generated.

The genomes of murine and human cytomegaloviruses (MCMV and HCMV; Messerle et al., 1997; Borst et al., 1999), herpes simplex virus type 1 (HSV-1; Suter et al., 1998), pseudorabies virus (PrV; Smith et al., 1999,2000), and Epstein-Barr virus (EBV; Delecluse et al., 1998) have been cloned as infectious BACs using this technique.

The aim of this study was to provide a basis for fast and efficient production of MDV-1 recombinants by cloning of the complete 180 kbp genome in *Escherichia coli.* Infectious MDV-1 was readily recovered after transfection of cloned MDV-1 BAC DNA using CEF cells and MDV-1 BACs were stable after several rounds of bacterial growth or serial propagation in CEF cells.

Lastly, because one-step deletion of an essential MDV-1 gene in *Escherichia coli* was possible, the system may have great potential to facilitate future analysis of essential and nonessential MDV-1 genes and serve as a tool for production of biologically safe modified live virus and/or DNA vaccines.

### Materials and Methods:

Virus and cells. Primary or secondary chicken embryo fibroblasts (CEF) or quail muscle (QM7) cells were maintained in Dulbecco's modified essential medium (DMEM) supplemented with 5 to 10 % fetal calf serum (FCS). MDV-1 strain 584Ap80C was kindly provided by Dr. Richard Witter, ADOL, East Lansing, Michigan, U.S.A. Strain 584Ap80C represents an avirulent cell culture passaged descendant of vv+ strain 584A (Witter, 1997) and was grown on primary or secondary CEF cells as previously described (Osterrieder, 1999). QM7 cells were tested for the absence of MDV-1 sequences by PCR and Southern blot hybridization targeting different regions of the genome before they were used for propagation of MDV-1 (Zelnik and Osterrieder, unpublished). Virus growth curves were done as described with slight modifications (Parcells et al., 1994). Briefly, 100 plaque-forming units (p.f.u.) were used to infect 2 X 10⁶ freshly seeded CEF cells. At various times after infection (0, 12, 24, 48, 72, 96, 120 hr), infected cells were trypsinized and titrated on fresh CEF cells. Numbers of plaques were determined and results represent means of two independent experiments.

A QM7 cell line constitutively expressing MDV-1 gB was obtained by transfection of 1 X 10⁶ QM7 cells with 10 µg of pcMgB (Fig. 1), which is based on pcDNA3 (Invitrogen) and contains the MDV-1 gB gene from strain Rispens CVI988 under the control of the human cytomegalovirus immediate early promoter. pcMgB-containing QM7 cells were selected in the presence of 1 mg/ml G418, and gB-expressing clones were identified using anti-gB monoclonal antibody (mab) 2K11 (kindly provided by Dr. Jean-Francois Vautherot, INRA, Tours, France). The resulting cell line expressing MDV-1 gB was termed MgB1.

Construction of MDV-1 BACs. MDV-1 DNA was purified from infected cells by sodium dodecyl sulfate-Proteinase K extraction as described earlier (Morgan et al., 1990). Plasmid pDS-pHA1 was constructed as follows. 2.1 and 3.1 kbp fragments on either side of the MDV-1 U_{S}2 gene (Fig. 1) were amplified by polymerase chain reaction (PCR) using standard primers containing appropriate restriction enzyme sites (Table 1), and both fragments were cloned into pTZ18R (Pharmacia-Amersham). The BAC vector containing the Eco-gpt gene under the control of the HCMV immediate early promoter was released from plasmid pHA1 (kindly provided by Dr. M. Messerle, LMU Munich, Germany; Messerle et al., 1997) and inserted into the *Pac*I sites introduced into both the 2.1 and 3.1 kbp fragment present in plasmid pDS (Fig. 1).

Primary CEF cells were co-transfected with 2 µg 584Ap80C DNA and 10 µg of pDS-pHA1. At 5 days after transfection, cells were plated on primary CEF cells in the presence of 250 µg/ml mycophenolic acid (MPA), 50 µg/ml xanthine and 100 µg/ml hypoxanthine. The MPA/xanthine/hypoxanthine selection was repeated for a total of four times. After complete cytopathic effect (cpe) had developed after the fourth round of selection, viral DNA was prepared from infected cells and 1 µg of infected-cell DNA was electroporated into DHB10 *Escherichia coli* cells. Colonies were detected from 16 hr after transfection on agar plates containing 30 µg/ml chloramphenicol (Sambrook et al., 1989). Single colonies were picked and BAC DNA was prepared from *Escherichia coli* following a standard alkaline lysis protocol (Sambrook et al., 1989). Large scale preparation of BAC DNA was performed by silica-based affinity chromatography using commercially available kits (Qiagen, Macherey & Nagel). Three MDV-1 584Ap80C BAC clones (BAC19, BAC20, BAC24) were chosen for further analysis.

Mutagenesis of MDV-1 BACs. For mutagenesis of cloned MDV-1 DNA in *Escherichia coli*, recE-catalyzed reactions promoting homologous recombination between linear DNA fragments, referred to as E/T cloning, was performed (Zhang et al., 1998; Narayanan et al., 1999). Plasmid pGETrec (kindly provided by Dr. Panos Ioannou, Murdoch Institute, Melbourne, Australia) harboring the recE, recT and bacteriophage 1 gam gene was transformed into BAC20-containing DH10B cells (Narayanan et al., 1999). After induction of recE, recT and gam by addition of 0.2 % arabinose, electrocompetent cells were prepared essentially as described (Narayanan). To delete the gB gene in BAC20, the kanamycin resistance gene (kan^{R}) of plasmid pEGFP-N1 (Clontech) was amplified by PCR. The designed primers contained 50 nucleotide homology arms bordering the desired deletion within gB and 20 nucleotides for amplification of kan^{R} (Table 1). The resulting 1.6 kbp fragment was purified from an agarose gel (Qiagen) and electroporated in pGETrec-containing BAC20 cells. Colonies harboring the cam^{R} and kan^{R} genes were identified on plates containing both antibiotics (Narayanan et al., 1999).

DNA analyses. BAC or viral 584Ap80C DNA was cleaved with *Eco*RI, *Bam*HI, *Bgl*II or *Stu*I and separated on 0.8 % agarose gels. DNA fragments were transferred to positively charged Nylon membranes (Pharmacia-Amersham) and Southern blot hybridization was performed using Digoxigenin-labeled BAC19 DNA or individual *Bam*HI fragments of MDV-1 strain GA (Fukuchi et al., 1991; Osterrieder, 1999).

In addition, a gB-specific probe from plasmid pcgB and a probe harboring the kan^{R} gene were prepared for analysis of gB-negative MDV-1 BAC. Chemoluminescent detection of DNA hybrids using CSPD™ was done according to the manufacturer's instruction (Roche Biochemicals).

Indirect immunofluorescence. For indirect immunofluorescence analyses (IIF), cells were grown on 6- or 24-well plates (Greiner) or on glass coverslips, and subsequently infected where indicated. Cells were fixed with 90% acetone at various times after infection or transfection, and IIF was done exactly as described(Meindl and Osterrieder, 1999). Samples were analyzed by fluorescence microscopy or confocal laser scanning microscopy (CLSM). The antibodies used were anti-gB mab 2K11, anti-pp38 mab H19 (kindly provided by Dr. Lucy Lee, ADOL, East Lansing, MI) or a convalescent serum from a chicken infected with MDV-1 (MDSI).

### Results:

Construction and analysis of BACs containing complete MDV-1 genomes. One million primary CEF were infected with 1 X 10⁴ p.f.u. of MDV-1 strain, i.e. infected cells were mixed with uninfected cells. After complete cytopathic effect had developed, DNA was prepared from infected cells and 2 µg viral DNA were transfected into 1 X 10⁶ primary CEF cells together with 10 µg of pDS-pHA1 plasmid DNA. Five days after transfection, cells were co-seeded with fresh CEF and overlaid with selection medium.

This procedure was repeated for a total of four times. Finally, DNA from recombinant MDV-1 that were able to grow in the presence of MPA/xanthine/hypoxanthine was isolated and subjected to Southern blot analysis using labeled pHAl as a probe. It could be demonstrated that a portion of the viral DNA contained inserted F plasmid sequences (data not shown). One microgram of this viral DNA was used to transform *Escherichia coli* DH10B cells. Transformed bacteria were plated on agar containing 30 µg/ml chloramphenicol and single colonies were picked. DNA of bacterial colonies was extracted by standard plasmid preparation procedures (Sambrook et al., 1989) and run on 0.8 % agarose gels.

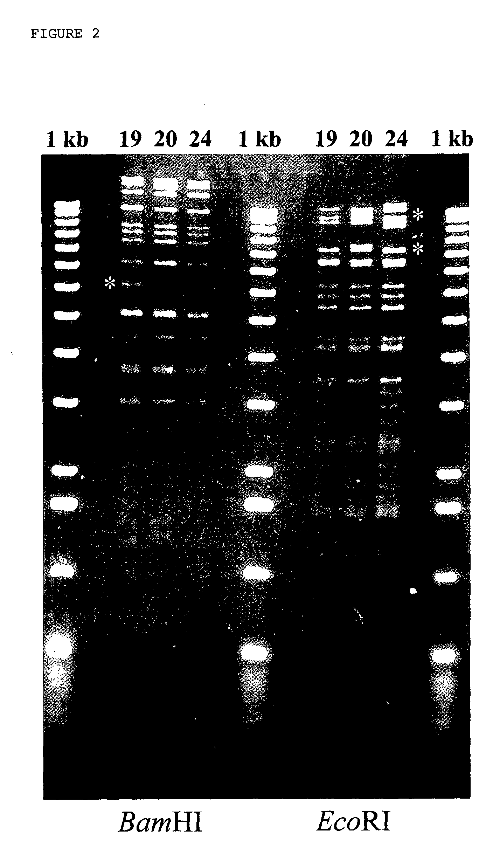

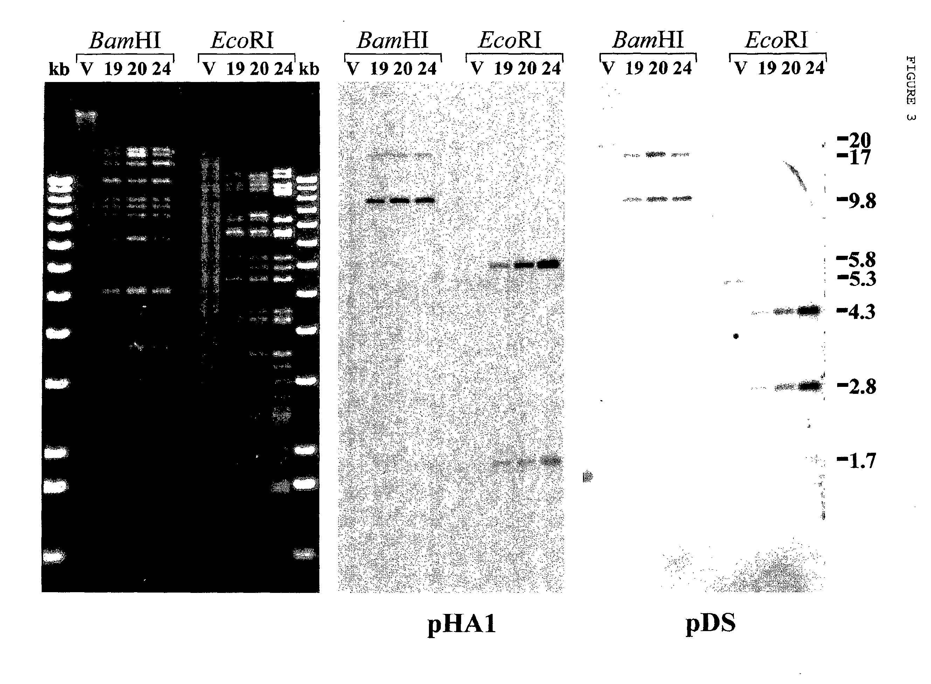

Several of the bacterial colonies were shown to contain high molecular weight extrachromosomal DNA, and three of the clones (BAC19, BAC20 and BAC24) were chosen for further analysis (Fig. 2). To further characterize the isolated BAC clones, Southern blot analysis of 584Ap80C and BAC DNA after cleavage with *Bam*HI or *Eco*RI with labeled BAC19 DNA as a probe was performed. It could be demonstrated that BAC19, BAC20, and BAC24 DNA exhibited almost identical restriction enzyme fragment patterns when compared to that of the parental 584Ap80C (Fig. 3A and B). Two notable exceptions, however, were readily recognized. The 20 kbp *Bam*HI-A fragment present in 584Ap80C DNA was absent in all analyzed BAC clones. Instead, fragments of 16 and 10 kbp in size were detected in DNA of BAC19, BAC20 and BAC24 (Fig. 3B). These two bands represented the enlarged *Bam*HI-A fragment in which by virtue of insertion of the F plasmid and deletion of U_{S}2 sequences an additional *Bam*HI site was introduced (Fig. 1).

In EcoRI-digested BAC DNA, one additional band of 5.8 kbp (BAC sequences) and minor alterations in sizes of fragments caused by the deletion of the U_{S}2 gene were observed (Fig. 1 and 3B). The correct insertion of the BAC sequences in the various clones was further analyzed by Southern blot hybridizations using labeled inserts of plasmid pDS or pHA1 as a probe, and the expected reaction pattern in *Bam*HI- or *Eco*RI-digested DNA was observed. In *Bam*HI-digested BAC DNAs, 16 and 10 kbp *Bam*HI fragments specifically reacted with the pDS probe whereas only the 10 kbp fragment was reactive with a probe derived from plasmid pHA1 (Fig. 1; Fig. 3C and D).

In *Eco*RI-digested BAC19, BAC20 or BAC24 DNA, fragments of 4.3, 2.8 and 1.7 kbp specifically reacted with the pDS probe, whereas 5.8 and 1.7 kbp fragments specifically hybridized with the pHA1 probe (Fig. 1, Fig. 3C and D). These fragments exactly corresponded to those predicted after insertion of the pHA1 sequences (Fig. 1), and it was concluded that the F plasmid sequences were correctly inserted instead of the U_{S}2 ORF in all MDV-1 BACs analyzed. In addition, some variation in banding patterns of BAC19, BAC20, and BAC24 was noted in either *Bam*HI or *Eco*RI digested DNA, e.g. an additional band of approximately 6.2 kbp in *Bam*HI-digested BAC19 DNA or additional bands in *Eco*RI-digested DNA of BAC20 and BAC24 (Fig. 2, 3A and B). To address the question of the observed size variations of individual restriction enzyme fragments, hybridization with labeled *Bam*HI-D fragment was performed because size variations in the terminal and internal repeats of the unique long region (TRL and IRL) are common.

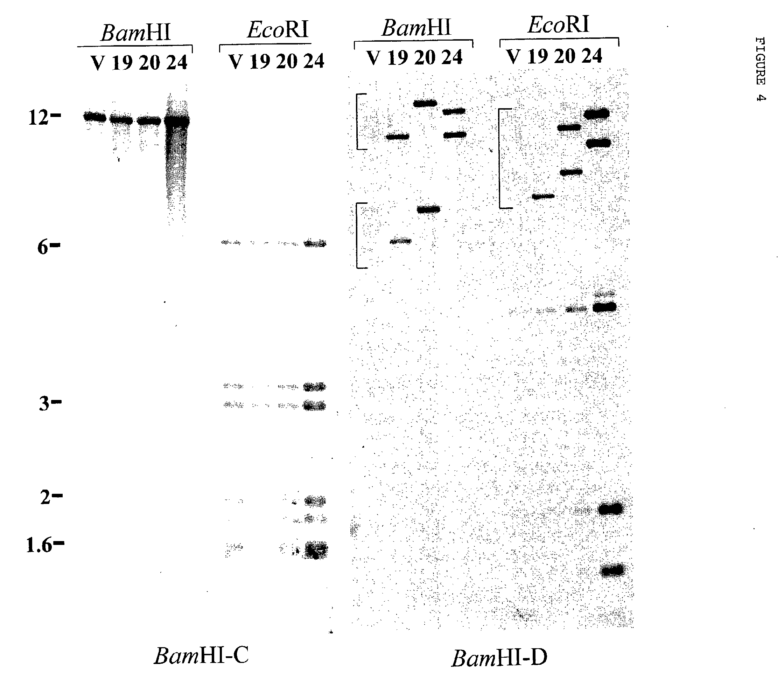

It was shown by Southern blotting that the additional fragments observed in either *Bam*HI- or *Eco*RI-digested DNA of BAC19, BAC20, or BAC24 resulted indeed from variations in TRL and IRL. Whereas two broad smears with the *Bam*HI-D probe were detected in viral 584Ap80C DNA digested with *Bam*HI which ranged from approximately 9 to 15 kbp and from 4 to 8 kbp (corresponding to the *Bam*HI-D and -H fragments of virulent MDV-1, respectively; Fig. 1), distinct but different bands were observed in all BAC clones analysed (Fig. 4). All other restriction enzyme fragments of the different BAC clones appeared to be identical with those of viral 584Ap80C DNA. This was confirmed by using several other labeled *Bam*HI fragments as probes, including *Bam*HI-A, -B, -C and -I₂ fragments (data for the BamHI-C probe are examplarily shown in Fig. 4).

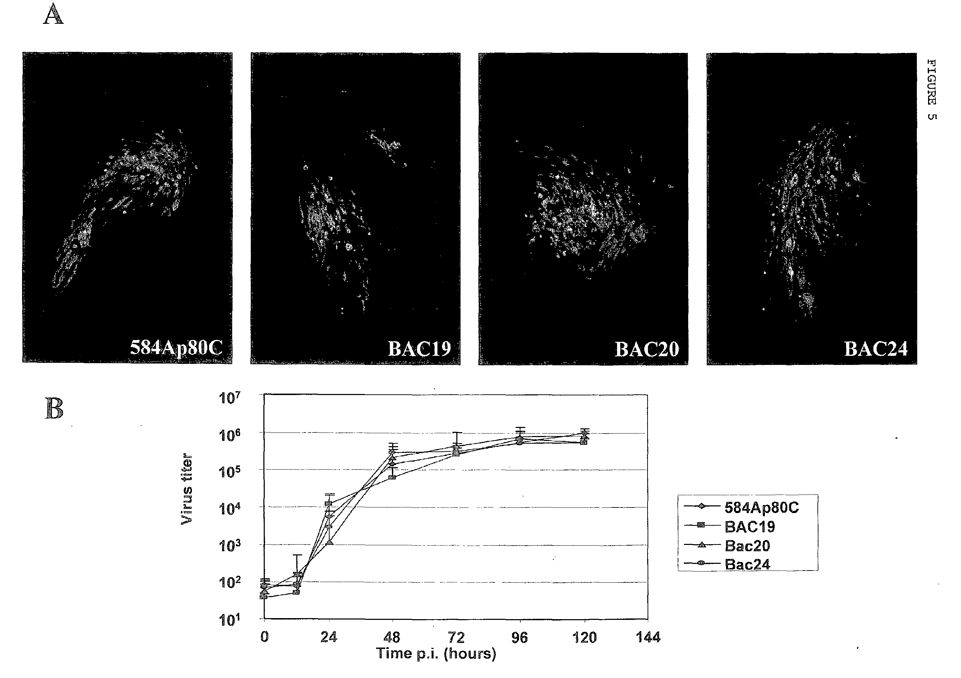

Reconstitution of infectious MDV-1 from cloned DNA. DNA of BAC19, BAC20 or BAC24 was transfected into primary CEF. At 3 to 7 days after transfection, MDV-1 specific virus plaques appeared as demonstrated by IIF using anti-MDV-1 gB mab. MDV-1 rescued after transfection of the various BACs was then co-seeded with fresh CEF and sizes of plaques were compared to those induced by parental 584Ap80C. As examplarily shown for plaques stained on day 2 p.i., no appreciable differences in plaque sizes between recombinant and parental viruses were detected (Fig. 5A).

To further characterize the biological properties of MDV-1 recovered after BAC transfection, growth kinetics of these viruses were compared to that of parental 584Ap80C. In case of BACs, virus recovered at day 5 after transfection were used to infect fresh CEF cells seeded on 6-well plates (50 p.f.u. of virus were used to infect one well containing 1 X 10⁶ cells). Similarly, 50 p.f.u. of 584Ap80C were used to infect fresh CEF in the same way. At various times p.i., virus was harvested and titrated by co-seeding 10-fold virus dilutions with fresh CEF cells. The results of these experiments are summarized in Fig. 5B.

It could be demonstrated that all MDV-1 BACs tested exhibited growth characteristics that were almost identical to those of parental 584Ap80C (Fig. 5B). Maximal titers were reached at 72 hr p.i. and remained virtually constant until the end of the observation period at 120 hr p.i. From the plaque sizes and growth characteristics we concluded that the biological properties of MDV-1 BACs *in vitro* were virtually indistinguishable from those of the parental strain.

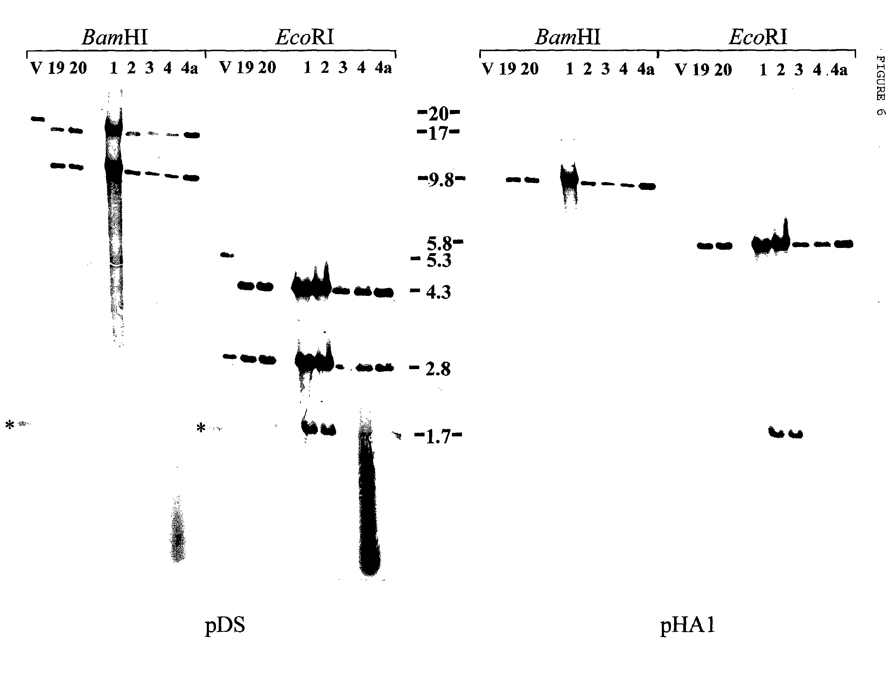

To ascertain stability of the BAC-derived viruses, progeny of BAC transfections of BAC19 and BAC 20 was passaged four times and viral DNA was prepared. Viral DNA was cleaved with *Bam*HI or *Eco*RI, separated by 0.8% agarose gel electrophoresis, and transferred to Nylon membranes. Hybridization was performed using the pDS or the pHA1 probe. Similar DNA fragments as described above were observed and the banding pattern did not change with serial passage of transfection progeny as analyzed with the two probes (Fig. 6). From these results we concluded that F plasmid-derived sequences remained stably inserted within the 584Ap80C genomes recovered from individual MDV-1 BAC clones even after serial passage in CEF cells.

However, as shown by hybridization with the *Bam*HI-D fragment and PCR analysis, variability of the 132-bp repeat sequences was restored and a diffuse smear of reactive bands was observed in *Bam*HI- or EcoRI-cleaved DNA of transfection progeny already after the first virus passage (data not shown).

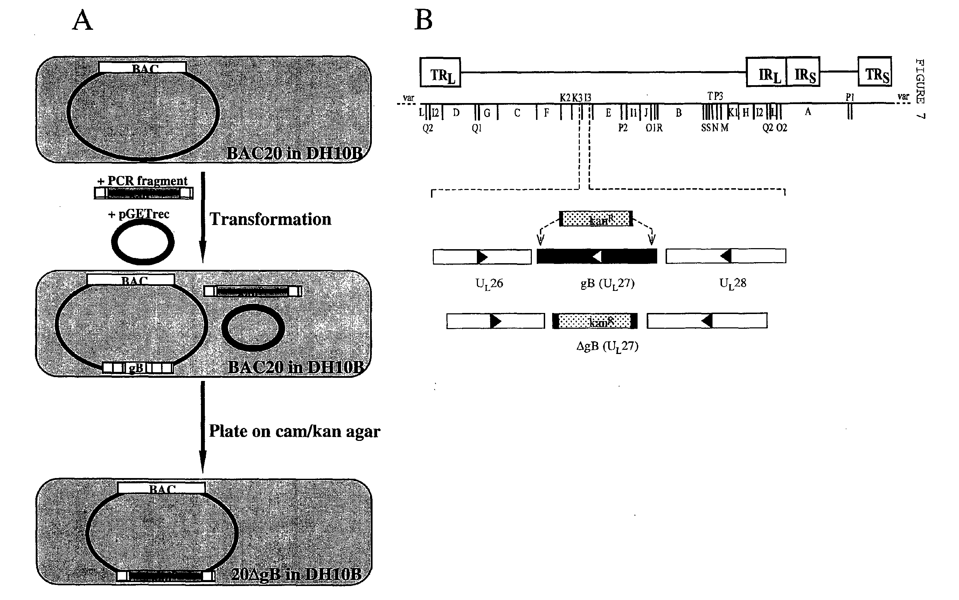

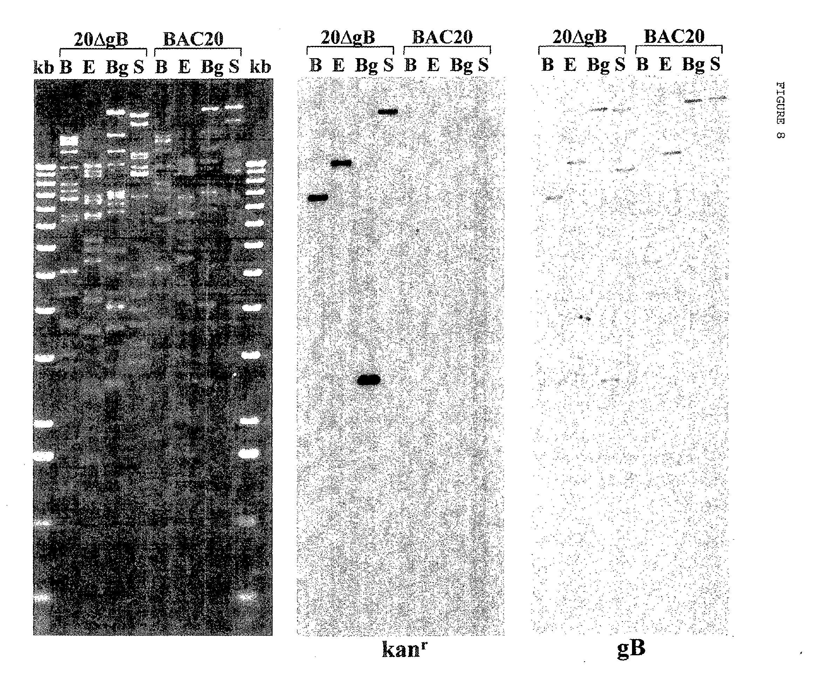

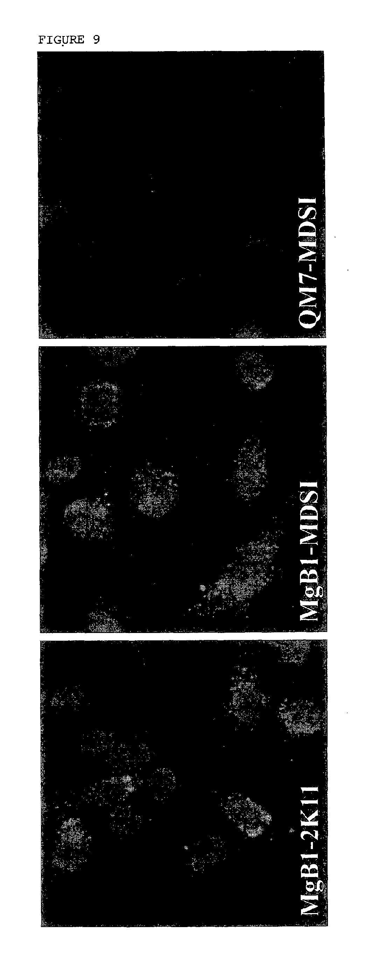

Mutagenesis of BAC20 and deletion of gB-encoding sequences. In the next experiments, a recently developed method for mutagenesis of BACs was applied to remove 2.3 kbp of the 2.8 kbp gB gene from BAC20 (Fig. 7). After transformation of plasmid pGETrec (Narayanan) into BAC20-containing DH10B, the kan^{R} gene was amplified with primers that allowed homologous recombination with MDV-1 gB sequences (Table 1; Fig. 8) and electroporated into BAC20-pGETrec cells. Bacteria were plated on LB agar containing chloramphenicol and kanamycin, and double resistant colonies were picked. After DNA isolation of individual colonies, Southern blot analysis of recombinant BAC20 harboring a deletion within the gB gene (20DgB) was performed. A kan^{R}- and a gB-specific probe detected fragments of 20DgB after cleavage with *Bam*HI, *Eco*RI, *Bgl*II or *Stu*I that were in perfect agreement with those calculated after insertion of the kan^{R} resistance gene into gB-encoding sequences (Fig. 9). It was noted that - as reported previously-pGETrec which confers ampicillin resistance was easily lost from *Escherichia coli* cells grown in the absence of the antibiotic (Fig. 9). From these results we concluded that the gB open reading frame was almost completely removed from 20DgB.

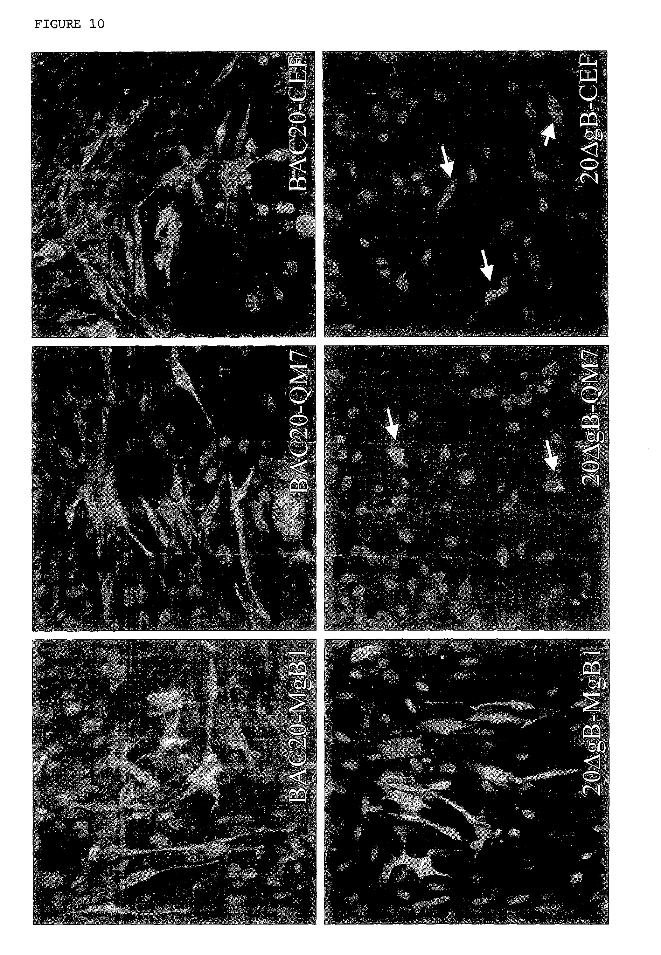

Analysis of gB-negative MDV-1 reconstituted from 20DgB. Because gB is essential for growth of all herpesviruses analyzed to date (reviewed in Pereira), a QM7 cell line which expressed MDV-1 gB under the control of the HCMV immediate early promoter was generated. Indirect immunofluorescence analyses demonstrated that virtually every cell of cell line MgB1 constitutively expressed MDV-1 gB as demonstrated using mab 2K11 or a convalescent chicken serum (MDSI) (Fig. 10). To analyze growth of BAC20 and 20DgB in various cell lines, DNA was prepared and used to transfect CEF, QM7 or MgB1 cells. At 3 to 5 days after transfection, virus plaques were observed in all cells transfected with BAC20 (Fig. 10).

However, after transfection of 20DgB DNA, plaques were observed in gB-expressing MgB1 cells only (Fig. 11). In CEF and QM7 cells transfected with 20DgB, single cells expressed the early pp38 gene as demonstrated by reactivity with mab H19 (Lee et al., but plaque formation was inhibited (Fig. 11). These results of gB being essential for MDV-1 cell-to-cell spread *in vitro* were confirmed by co-seeding 20DgB-infected MgB1 cells with CEF, QM7 or fresh MgB1 cells. As shown after primary transfection, plaque formation was only observed after co-seeding with gB-expressing cells (Table 2). From these results we concluded, that MDV-1 gB is essentially required for cell-to-cell spread of MDV-1 in cultured cells.

Although Marek's disease virus is an important pathogen of chickens that causes T cell tumors and high mortality in infected animals, little is known about the function of individual genes and gene products in the lytic, latent or tumor phase of the infection. Analysis of MDV-1 genes and gene products has been greatly impaired for two main reasons. Firstly, cultured cells infected with MDV-1 do not yield free infectious virus, and secondly, efficient growth of MDV-1 in cultured cells is restricted to primary or secondary chicken embryo fibroblasts.

Hence, mutagenesis using conventional homologous recombination used to mutagenize other *Alphaherpesvirinae* is laborious, time-consuming and requires constant supply of primary cells. While mutagenesis of HSV and PrV is certainly facilitated by using the BAC technology, conventional mutagenesis relying on homologous recombination in eukaryotic cells represents a standard technique for these two viruses and numerous mutant viruses have been generated. In contrast, for mutagenesis of MDV-1 BAC cloning and mutagenesis is a major advantage. Once the MDV-1 genome is cloned as a BAC and can be stably maintained in *Escherichia coli,* generation of mutants and analyses of essential genes should be relatively easy. In fact, it was possible to clone the complete genome of strain 584Ap80C as an infectious BAC. Strain 584Ap80C is a descendant of the very virulent plus (vv+) MDV-1 strain 584A after 80 serial passages on CEF cells (Witter, 1997). Analysis of the cloned MDV-1 genomes present in BAC19, BAC20 and BAC24 demonstrated that variations of restriction enzyme patterns were obvious.

This heterogeneity could be attributed to variations in the *Bam*HI-D and -H fragments. It is known that varying numbers of 132-bp tandem repeats are present in various MDV-1 strains and that the number of repeats increases after serial passage in cultured cells (Maotani, Silva, Fukuchi). In addition, the number of the tandem 132-bp repeats was associated with a loss of oncogenicity because a constant number of these units was demonstrated in virulent strains (Fukuchi et al., 1985; Bradley et al., 1989), although recent work on the widely used Rispens CVI 988 vaccine strain demonstrated that there might be no direct correlation of small numbers of the 132-bp repeats and virulence. In case of MDV-1 strain 584Ap80C, hybridization of restriction enzyme digested viral DNA with the *Bam*HI-D fragment yielded diffuse banding patterns indicating a variable number of repeats present in the virus population. In contrast, only single strongly reactive bands were identified in each of the BAC clones with the same probe. However, the sizes of reactive bands after cleavage with *Bam*HI or *Eco*RI varied between BAC19, BAC20 and BAC24 indicating that genomes containing different numbers of the 132-bp repeats had been cloned. This interpretation was substantiated by PCR analyses targeting the 132-bp repeats. Whereas the typical ladder-like appearance of PCR products was obtained with DNA of 584Ap80C (Becker et al., 1993), distinct bands were amplified from cloned viral DNA in case of BAC19, BAC20 or BAC24.

It was therefore concluded that the variable restriction enzyme patterns of the different BAC clones resulted from various numbers of tandem 132-bp repeats present in the individual clones which did not influence the infectivity of the cloned DNA because infectious virus was recovered after transfection of DNA isolated from each of the different BAC clones.

After cloning of the complete MDV-1 genome and proof of infectivity of cloned MDV-1 DNA, a recently developed mutagenesis system in which a linear DNA fragment can be recombined into bacteria-resident DNA and which is catalyzed by recE (Narayanan, muyrers) was used to delete gB encoding sequences of BAC20. The mutagenesis is based on recE, recT and the recB/C-suppressing λ gam gene present on plasmid pGETrec (Narayanan et al., 1999).

The big advantages of the system that was used for the first time to manipulate a virus genome are (i) that only 30 to 50 bp homology arms are needed to target a specific sequence to be deleted, i.e. deletion of any open reading frame can be achieved without the need to clone recombination cassettes, (ii) the method is very fast, and (iii) that the pGETrec vector conferring the mutagenesis system and expressing ampicillin resistance is rapidly lost from bacterial cells in the absence of ampicillin. After electroporation of the gB knockout PCR product into pGETrec-containing BAC20 cells, between 10 and 30 cam^{R} and kan^{R} double-resistant colonies were obtained. One of the colonies was termed 20DgB-1 and chosen for further analyses because it had lost pGETrec immediately after plating on agar containing chloramphenicol and kanamycin.

Southern blot analyses demonstrated successful deletion of the gB gene and the insertion of the kan^{R} gene in 20DgB-1. MDV-1 recovered after transfection of CEF cells with 20DgB-1 was unable to spread from infected cells to neighboring cells indicating that MDV-1 gB like its counterparts in other herpesviruses is essential for cell-to-cell spread of infectivity. Because MDV-1 is highly cell-associated in cultured cells and does not release infectious virus to the culture medium, we were not able to investigate a possible role of MDV-1 gB in virus entry. The generated gB mutant represents the first example of an MDV-1 with deletion of an essential gene and demonstrates the power of the BAC cloning and mutagenesis system which is especially useful in case of MDV-1. Using MDV-1 BAC and the permanent cell line QM7 which allows MDV-1 propagation and which - unlike quail fibroblast QT35 cell line- does not harbor MDV-1 sequences (Zelnik et al., unpublished), represents an excellent combination to thoroughly analyze essential MDV-1 genes. In addition, comparative analyses on gene functions of various *Alphaherpesvirinae* can now include MDV-1 and allows studies on very distantly related members, such as VZV or BHV-4 of one viral family.

With the cloned genomes as provided herein in at hand, a detailed further assessment of lytic, latent and tumor-inducing genes is provided for virus in which genetic manipulations used to be very restricted.

### References:

1. **Anderson, A.S., Parcells, M.S., and R.W. Morgan.** 1998. The glycoprotein D (US6) homolog is not essential for oncogenicity or horizontal transmission of Marek's disease virus. *J Virol.* **72**: 2548-2553.
2. **Becker, Y., E. Tabor, Y. Asher, I. Davidson, M. Malkinson, and R.L. Witter**. 1993. PCR detection of amplified 132 bp repeats in Marek's disease virus type 1 (MDV-1) DNA can serve as an indicator for critical genomic rearrangement leading to the attenuation of virus virulence. *Virus Genes* **7**: 277-287.
3. **Borst, E.M., G. Hahn, U.H. Koszinowski, and M. Messerle**. 1999. Cloning of the human cytomegalovirus (HCMV) genome as an infectious bacterial artificial chromosome in Escherichia coli: a new approach for construction of HCMV mutants. *J. Virol.* **73**: 8320-8329.
4. **Bradley, G., M. Hayashi, G. Lancz, A. Tanaka, and M. Nonoyama**. 1989. Structure of the Marek's Disease virus BamHI-H gene family: Genes of putative importance for tumor induction. *J. Virol.* **63**: 2534-2542.
5. **Bradley, G., G. Lancz, A. Tanaka, and M. Nonoyama.** 1989. Loss of Marek's disease virus tumorigenicity is associated with truncation of RNAs transcribed within BamHI-H. *J. Virol.* **63**: 4129-4235.
6. **Brune, W., C. Menard, U. Hobom, S. Odenbreit, M. Messerle, and U.H. Koszinowski**. 1999. Rapid identification of essential and nonessential herpesvirus genes by direct transposon mutagenesis. *Nat. Biotechnol.* **17**:360-364.
7. **Brunovskis, P., and L.F. Velicer**. 1995. The Marek's disease virus (MDV) unique short region: alphaherpesvirus-homologous, fowlpox virus-homologous, and MDV-specific genes. *Virology* **206**: 324-38.
8. **Cantello, J.L., A.S. Anderson, A. Francesconi, and R.W. Morgan**. 1991. Isolation of a Marek's disease virus (MDV) recombinant containing the lacZ gene of Escherichia coli stably inserted within the MDV US2 gene. J. *Virol.* **65**:1584-1588.
9. **Cui, Z.Z., D. Yan, and L.F. Lee**. 1990. Marek's disease virus gene clones encoding virus-specific phosphorylated polypeptides and serological characterization of fusion proteins. *Virus Genes* **3**:309-322.
10. **Delecluse, H.J., T. Hilsendegen, D. Pich, R. Zeidler, and W. Hammerschmidt.** 1998. Propagation and recovery of intact, infectious Epstein-Barr virus from prokaryotic to human cells. *Proc. Natl. Acad. Sci. U.S.A.* **95**: 8245-8250.
11. **Fuckuchi, K., M. Sudo, Y.S. Lee, A. Tanaka, and M. Nonoyama.** 1984. Structure of Marek's disease virus DNA: detailed restriction enzyme map. *J. Virol.* **51**: 102-109.
12. **Lee, L.F., P. Wu, D. Sui, D. Ren. J. Kamil, H.J. Kung, and R. L. Witter**. 2000. The complete unique long sequence and the overall genomic organization of the GA strain of Marek's disease virus. *Proc. Natl. Acad. Sci. U.S.A.* **97**:6091-6096.
13. **Maotani, K., K. Kanamori, K. Ikuta, S. Ueda, S. Kato, and K. Hirai.** 1986. Amplification of a tandem repeat within inverted repeats of Marek's disease virus DNA during serial in vitro passage. *J. Virol.* **58**: 657-659.
14. **Meindl, A. and N. Osterrieder**. 1999. The equine herpesvirus type 1 Us2 homolog encodes a nonessential membrane-associated virion component. *J. Virol.* **73**, 3430-3437.
15. **Messerle, M., I. Crnkovic, W. Hammerschmidt, H. Ziegler, and U.H. Koszinowski**. 1997. Cloning and mutagenesis of a herpesvirus genome as an infectious bacterial artificial chromosome. *Proc. Natl. Acad. Sci. U.S.A.* **94**: 14759-14763.
16. **Morgan, R.W., J.L. Cantello, and C.H. McDermott**. 1990. Transfection of chicken embryo fibroblasts with Marek's disease virus DNA. *Avian Dis.* **34**: 345-351.
17. **Muyrers, J.P., Y. Zhang, G. Testa, and A.F. Stewart**. 1999. Rapid modification of bacterial artificial chromosomes by ET-recombination. *Nucleic Acids Res.* **27**: 1555-1557.
18. **Narayanan, K., R. Williamson, Y. Zhang, A.F. Stewart, and P.A. Ioannou**. 1999. Efficient and precise engineering of a 200 kb beta-globin human/bacterial artificial chromosome in E. coli DH10B using an inducible homologous recombination system. *Gene Ther.* **6**: 442-447.
19. **Osterrieder, N.** 1999. Sequence and initial characterization of the U_{L}10 (glycoprotein M) and U_{L}11 homologous genes of serotype 1 Marek's Disease Virus. *Arch. Virol.* **144**, 1853-63.
20. **Osterrieder, N., A. Neubauer, C. Brandmüller, B. Braun, O.-R. Kaaden, and J.D. Baines.** 1996. The equine herpesvirus type 1 glycoprotein gp21/22a, the herpes simplex virus type 1 gM-homolog, is involved in virus penetration and cell-to-cell spread of virions. *J. Virol* **70**: 4110-4115.
21. **Parcells, M.S., A.S. Anderson, J.L. Cantello, and R.W. Morgan**.1994. Characterization of Marek's disease virus insertion and deletion mutants that lack US1 (ICP22 homolog), US10, and/or US2 and neighboring short-component open reading frames. *J. Virol.* **68**: 8239-8253.
22. **Parcells, M.S., A.S. Anderson, and R.W. Morgan**. 1994. Retention of oncogenicity by a Marek's disease virus mutant lacking six unique short region genes. *J. Virol.* **69**: 7888-7898.
23. **Parcells, M.S., A.S. Anderson, and R.W. Morgan**. 1994. Characterization of a Marek's disease virus mutant containing a lacZ insertion in the US6 (gD) homologue gene. Virus Genes 9:5-13.
24. **Payne, L.N.** 1985. Pathology. In: LN Payne (ed) Marek's Disease. Kluwer Academic Publishers, Hingham, MA, U.S.A., pp. 43-76.
25. **Pereira, L.** 1994. Function of glycoprotein B homologues of the family herpesviridae. *Infect. Agents Dis.* **3**: 9-28.
26. **Ross, N.L.J., Binns, M.M., and J. Pastorek**. 1991. DNA sequence and organization of genes in a 5.5 kbp EcoRI fragment mapping in the short unique segment of Marek's disease virus (strain RB1B). J. *Gen. Virol.* **72**: 949-954.
27. **Sakaguchi, M., T. Urakawa, Y. Hirayama, N. Miki, M. Yamamoto, G.S. Zhu, and K. Hirai**.1993. Marek's disease virus protein kinase gene identified within the short unique region of the viral genome is not essential for viral replication in cell culture and vaccine-induced immunity in chickens. *Virology* **195**: 140-1488.
28. **Sambrook, J., D.F. Fritsch, and T. Maniatis**. 1989. Molecular Cloning: A Laboratory Manual. Cold Spring Harbor Laboratory Press, Cold Spring Harbor.
29. **Schat, K.A.** 1985. Characteristics of the virus. In: LN Payne (ed) Marek's Disease. Kluwer Academic Publishers, Hingham, MA, U.S.A., pp. 77-112.
30. **Schat, K.A., B.J. van Iddekinge, H. Boerrigter, P.H. O'Connell, and G. Koch**. 1998. Open reading frame L1 of Marek's disease herpesvirus is not essential for in vitro and in vivo virus replication and establishment of latency. *J. Gen. Virol.* **79**: 841-849.
31. **Silva, R.F., and R.L. Witter**. 1985. Genomic expansion of Marek's disease virus DNA is associated with serial in vitro passage. *J. Virol.* **54**: 690-696.
32. **Smith G.A., and L.W. Enquist**. 1999. Construction and transposon mutagenesis in Escherichia coli of a full-length infectious clone of pseudorabies virus, an alphaherpesvirus. *J. Virol.* **73**: 6405-6414.
33. **Smith, G.A., and L.W. Enquist**. 2000. A self-recombining bacterial artificial chromosome and its application for analysis of herpesvirus pathogenesis. *Proc. Natl. Acad. Sci. U.S.A.* **97**: 4873-4878.
34. **Suter, M., A.M. Lew, P. Grob, G.J. Adema, M. Ackermann, K. Shortman, and C. Fraefel**. 1999. BAC-VAC, a novel generation of (DNA) vaccines: A bacterial artificial chromosome (BAC) containing a replication-competent, packaging-defective virus genome induces protective immunity against herpes simplex virus 1. *Proc. Natl. Acad. Sci. U.S.A.* **96**: 12697-12702.
35. **van Iddekinge, B.J., L. Stenzler, K.A. Schat, H. Boerrigter, and G. Koch**. 1999. Genome analysis of Marek's disease virus strain CVI-988: effect of cell culture passage on the inverted repeat regions. *Avian Dis.* **43**:182-188.
36. **van Regenmortel, M.H.V., C.M. Fauquet, D.H.L. Bishop, E. Carstens, M.K. Estes, S. Lemon, J. Maniloff, M.A. Mayo, D. McGeoch, C.R. Pringle, and R.B. Wickner (eds).** 1999. Virus Taxonomy. Seventh Report of the International Committee on Taxonomy of Viruses. Academic Press, New York, San Diego.
37. **Wagner, M., S. Jonjic, U.H. Koszinowski, and M. Messerle**. 1999. Systematic excision of vector sequences from the BAC-cloned herpesvirus genome during virus reconstitution. *J. Virol.* **73**:7056-7060.
38. **Witter, R.L.** 1985. Principles of vaccination. In: L.N. Payne (ed): Marek's Disease. Kluwer Academic Publishers, Hingham, MA, U.S.A., pp. 203-250.
39. **Witter R.L.** 1997. Increased virulence of Marek's disease virus field isolates. *Avian Dis.* **41**: 149-163.
40. **Witter, R.L., J.M. Sharma, and A.M. Fadly**. 1980. Pathogenicity of variant Marek's disease virus isolants in vaccinated and unvaccinated chickens. Avian Dis. 24: 210-232.
41. **Zhang, Y., F. Buchholz, J.P. Muyrers, and A.F. Stewart**. 1998. A new logic for DNA engineering using recombination in Escherichia coli. *Nature Genet.* **20**:123-128.

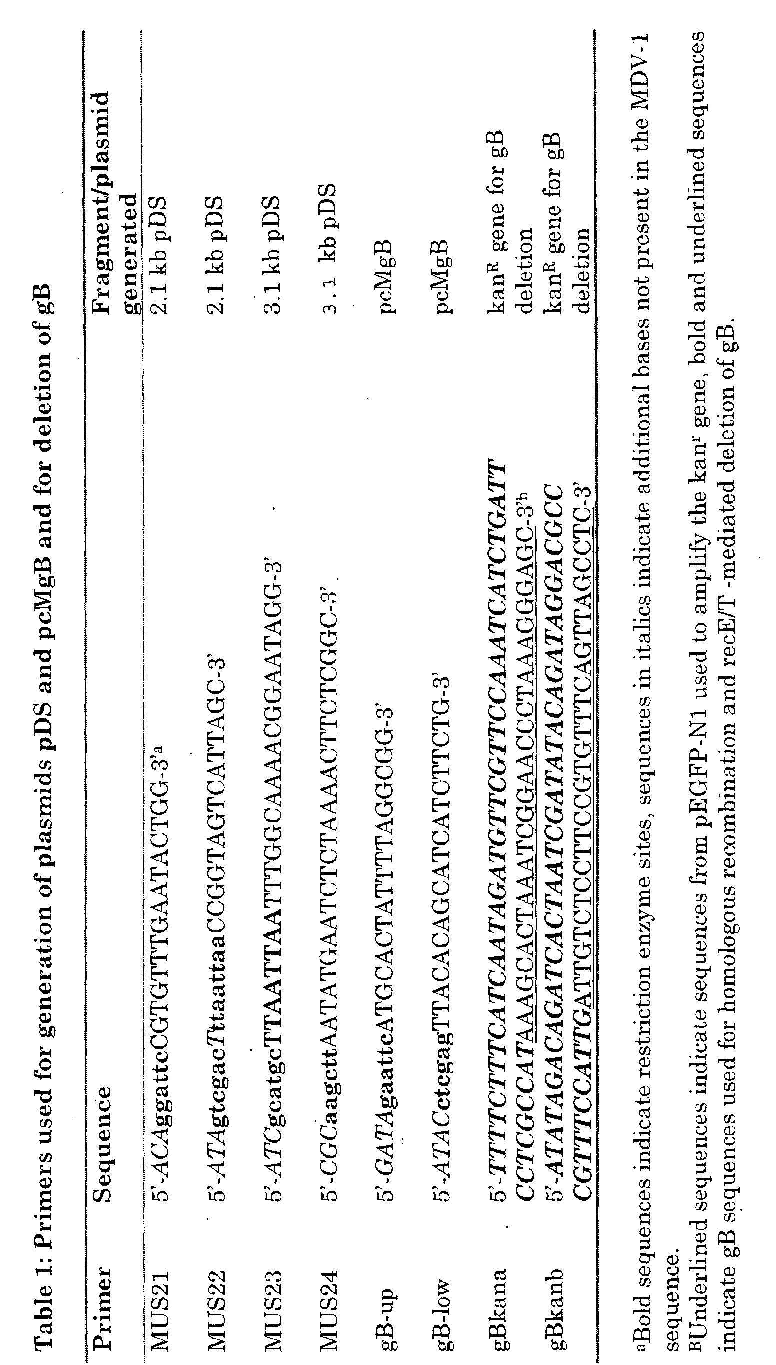

### Legends to Figures

**Figure 1** Schematic illustration of the cloning procedure to generate BACs harboring complete MDV-1 genomes. Shown is the organization of the approximately 180 kbp MDV-1 genome **(A)** and the *Bam*HI-restriction map **(B)** according to Fukuchi et al. (11). The unique short region (Us) and the ORFs located in the Us are shown (**C** and **D**). A 2.1 and a 3.1 kbp fragment bordering the Us2 gene (grey boxes) were amplified by PCR and cloned into plasmid pTZ18R to give rise to recombinant plasmid pDS. The 7.2 kbp BAC vector released from recombinant plasmid pHA1 (15 ) was inserted into pDS give plasmid pDS-pHA1 **(E)**. Restriction enzyme sites according to (2) are abbreviated: B = *Bam*HI, E = *Eco*RI, P = *Pst*I, Pa = *Pac*I, S = *Sal*I.

**Figure 2** Digitally scanned image of an ethidium bromide stained 0.8% agarose gel. DNA isolated from *Escherichia coli* DH10B clones BAC19, BAC20 and BAC24 was cleaved with *Bam*HI or *Eco*RI and separated. The restriction enzyme digests are flanked by the 1 kb ladder (Gibco-BRL). Asterisks indicate additional bands or size variations of individual fragments between the three BAC clones.

**Figure 3** Digitally scanned image of DNA of 584Ap80C (V), BAC19, BAC20 and BAC24 cleaved with *Bam*HI or EcoRI, separated by 0.8% agarose gel electrophoresis, and stained with ethidium bromide (left panel). After Southern transfer of DNA fragments to Nylon membranes, hybridization with Digoxigenin-labeled fragments released from plasmid pDS or pHA1 were performed. Size markers (1 kb ladder, Gibco-BRL) and sizes of reactive bands are given.

**Figure 4** Digitally scanned images of Southern blots to analyze size variations in BAC19, BAC20 and BAC24 DNA. Viral DNA of strain 584Ap80C (V) and individual BACs was cleaved with *Bam*HI or *Eco*RI and transferred to Nylon membranes. Sheets were incubated with Digoxigenin-labeled BAC19 DNA or labeled *Bam*HI-C or *Bam*HI-D fragments. Size markers (1 kb ladder, Gibco-BRL) are given. The smear-like appearance of bands in case of 584Ap80C DNA when hybridized with *Bam*HI-D sequences are indicated by brackets.

**Figure 5 (A)** IIF analysis of MDV-1 plaques after transfection of BAC19, BAC20 or BAC24 DNA. At 5 days after transfection, infected cells were fixed and subjected to indirect immunofluorescence using anti-gB mab 2K11. Detection of bound antibodies was performed with anti-mouse Alexa™ 488 (Molecular probes) and nuclei were counterstained with propidium iodide. Magnification = 400 X.
**(B)** Growth curves of MDV-1 strain 584A and various BACs. After infection of CEF cells with 100 p.f.u. of 584Ap80C or transfection progeny of BAC19, BAC20 or BAC24, virus titers were determined at the indicated times p.i. by co-seeding with fresh CEF cells. Plaques were counted after immunofluorescent staining with mab 2K11.

**Figure 6** Digitally scanned images of Southern blots to analyze the stability of BAC vector sequences in viruses recovered after transfection of BAC19 and BAC20. Transfection progeny was passaged for four times and after each passage, viral DNA was isolated. Virus DNA was cleaved with *Bam*HI or *Eco*RI, separated by 0.8% agarose gel electrophoresis and transferred to Nylon membranes. Southern blot hybridization was performed using Digoxigenin-labeled fragments of plasmids pDS or pHA1. Abbreviations: V = 584Ap80C, 19 = BAC19, 20 = BAC20. Passages 1 to 4 after transfection of BAC19 DNA are indicated by numbers 1 to 4. Passage 4 after transfection of BAC20 DNA was loaded in the last lane, respectively, and is indicated by 4a. Sizes of reactive fragments are given. Asterisks indicate the reactive 1.6 kb band of the marker (1 kb ladder, Gibco-BRL).

**Figure 7 (A)** Schematic illustration of mutagenesis of BAC20 to remove gB-encoding sequences. Recombinant plasmid pGETrec encoding L-arabinose-inducible recE, recT, and gam gene was transformed into BAC20-containing DH10B cells. After PCR amplification of the kan^{R} gene from plasmid pEGFP-N1 (Clontech) with primers that also contained 50 nt homology arms bordering the gB deletion, a 1.6 kbp PCR amplicon was electroporated into DH10B cells harboring BAC20 and pGETrec. Bacterial suspensions were plated on agar containing 30 µg/ml kanamycin and 30 µg/ml chloramphenicol. Double-resistant colonies were picked and subjected to further analysis.
**(B)** Schematic illustration of the location of the gB gene in MDV-1 and the deletion present in BAC 20DgB.

**Figure 8** Scanned image of an ethidium bromide stained 0.8% agarose gel containing BAC20 and 20DgB DNA cleaved with *Bam*HI, *Eco*RI, *Bgl*I, or *Stu*I and separated by 0.8% agarose gel electrophoresis (left panel). DNA fragments were transferred to nylon membranes and hybridized with a Digoxigenin-labeled kan^{R}- or gB-specific probe. Sizes of reactive DNA fragments are indicated. Abbreviations: B = *Bam*HI, E = EcoRI, Bg = *Bgl*I, S = Stul.

**Figure 9** Confocal laser scan analysis of MgB1 cells constitutively expressing MDV-1 gB. MgB1 or QM7 cells were seeded on glass coverslips and incubated with anti-gB mab 2K11 or convalescent chicken serum MDSI. Secondary antibodies were anti-mouse or anti-chicken IgG Alexa™ 488 conjugates (Molecular probes). Nuclei were counterstained with propidium iodide. Bar represents 10 µm.

**Figure 10** IIF analysis of MgB1, QM7 or CEF cells after transfection with BAC20 (upper panels) or 20DgB (lower panels). At 5 days after transfection, cells were fixed with acetone and incubated with anti-pp38 mab H19. The secondary antibody was anti-mouse IgG Alexa™ 488 (Molecular probes). Whereas MDV-1 plaques were observed on all cell lines after transfection of BAC20 DNA, viral plaques were only observed on MgB1 cells after transfection with 20DgB. Only single infected cells were observed on QM7 and CEF cells (arrowheads). Magnification = 400X.

## Claims

1. A vaccine directed against an infection caused by an essentially host cell associated herpesvirus said vaccine comprising a recombinant genome derived from said herpesvirus, said genome allowing recombination essentially free of said host cell.

2. A vaccine according to claim 1 said genome comprising a functional deletion in a gene essential for replication in and/or spread of said herpesvirus from a host cell.

3. A vaccine according to claim 1 or 2 said genome at least comprising a nucleic acid encoding an antigenic substance capable of eliciting an immune response against an infection of an individual with said herpesvirus.

4. A vaccine according to claims 1 to 3 said genome comprising a functional deletion in a gene essential for eliciting a marker immune response specific for said herpesvirus allowing discrimination between an individual vaccinated with said vaccine and an individual infected with said essentially cell associated herpesvirus.

5. A vaccine according to anyone of claims 1 to 4 said genome at least comprising a nucleic acid encoding a proteinaceous substance capable of modulating transcription and/or translation of a nucleic acid encoding an antigenic substance capable of eliciting an immune response against an infection of an individual with said herpesvirus.

6. A vaccine according to anyone of claims 1 to 5 wherein said genome comprises an essentially full-length copy derived from said herpesvirus.

7. A vaccine according to any one of above mentioned claims further provided with a nucleic acid at least encoding an antigenic substance of an additional pathogen.

8. A vaccine according to anyone of above mentioned claims wherein said herpesvirus comprises Marek's disease virus.

9. A vaccine according to claim 8 wherein said Marek's disease virus comprises serotype 1.

10. A vaccine according to claim 8 or 9 wherein said Marek's disease virus is derived from a virulent, a very virulent or a very virulent plus field virus.

11. A method for obtaining a recombinant viral genome from an essentially host cell associated herpesvirus comprising
transforming suitable bacterial cells with a plasmid that expresses *recE, recT* and the λ *gam* gene,
preparing circular viral DNA from lytically or latently infected cells infected with said virus,
providing linear bacterial artificial chromosome vector nucleic acid with sequences that allow homologous recombination of said vector nucleic acid with said viral DNA,
providing expression of *recE*, *recT and* the *gam* gene in said bacterial cells and electroporating said viral DNA with said linear vector nucleic acid into competent bacterial cells.

12. A recombinant viral genome derived from an herpesvirus that is essentially host cell associated said genome allowing recombination essentially free of said host cell.

13. A genome according to claim 12 at least comprising a replicative minigenome.

14. A genome according to claim 12 wherein said genome comprises an essentially full-length copy derived from said herpesvirus.

15. An essentially intracellular bacterium comprising a genome according to anyone of claims 12 to 14.

16. A bacterium according to claim 14 comprising a *Salmonella spp.*

17. Use of a genome according to anyone of claims 12 to 14 or a bacterium according to claim 15 or 16 for the preparation of a vaccine directed against a disease caused by an infection with an essentially host cell-associated herpesvirus.

18. A method for limiting the risks of an individual on acquiring or fully manifesting a disease caused by an infection with an essentially host cell-associated herpesvirus comprising administering to said individual a vaccine according to any one of claims 1 to 10, a genome according to anyone of claims 12 to 14 or a bacterium according to claim 15 or 16.

19. A method according to claim 18 wherein said individual is a bird.


### Annex to the application documents-subsequently filed sequences listing

SEQUENCE LISTING

<110> Lohmann Animal Health GMBH & Co. KG

<120> Vaccination against host cell-associated herpesviruses

<130> P53529EP00

<140> 00202757.1
<141> 2000-08-03

<160> 8

<170> PatentIn Ver. 2.1

<210> 1
<211> 25
<212> DNA
<213> Artificial Sequence

<220>
<223> Description of Artificial Sequence: primer MUS21

<400> 1
acaggattcc gtgtttgaat actgg 25

<210> 2
<211> 33
<212> DNA
<213> Artificial Sequence

<220>
<223> Description of Artificial Sequence: primer MUS22

<400> 2
atagtcgact ttaattaacc ggtagtcatt agc 33

<210> 3
<211> 36
<212> DNA
<213> Artificial Sequence

<220>
<223> Description of Artificial Sequence: primer MUS23

<400> 3
atcgcatgct taattaattt ggcaaaacgg aatagg 36

<210> 4

<211> 35
<212> DNA
<213> Artificial Sequence

<220>
<223> Description of Artificial Sequence: primer MUS24

<400> 4
cgcaagctta atatgaatct ctaaaacttc tcggc                35


<210> 5
<211> 28
<212> DNA
<213> Artificial Sequence

<220>
<223> Description of Artificial Sequence: primer gB-up

<400> 5
gatagaattc atgcactatt ttaggcgg                28


<210> 6
<211> 30
<212> DNA
<213> Artificial Sequence

<220>
<223> Description of Artificial Sequence: primer gB-low

<400> 6
atacctcgag ttacacagca tcatcttctg                30


<210> 7
<211> 80
<212> DNA
<213> Artificial Sequence

<220>
<223> Description of Artificial Sequence: primer gBkana

<400> 7
ttttctttca tcaatagatg ttcgttccaa atcatctgat tcctcgccat aaagcactaa 60
atcggaaccc taaagggagc                80


<210> 8
<211> 80
<212> DNA
<213> Artificial Sequence

<220>
<223> Description of Artificial Sequence: primer gBkanb

<400> 8
atatagacag atcactaatc gatatacaga taggacgccc gtttccattg attgtctcct 60
tccgtgtttc agttagcctc 80